# EUROPÄISCHE PATENTANMELDUNG

(11) **EP 2 386 647 A1**
(43) Veröffentlichungstag der Anmeldung: **16.11.2011**
(21) Anmeldenummer: 10004912.1
(22) Anmeldetag: 10.05.2010
(51) Int. Cl.: C12N 15/87, C12N 15/88

(54) **Verfahren zur Transfektion einer eurkaryotischen Zelle**

(71) Anmelder: Qiagen GmbH, 40724 Hilden (DE)
(72) Erfinder: Hahn, Peter, 51429 Bergisch Gladbach (DE); Grewe, Anja, 42699 Solingen (DE)

(57) **Zusammenfassung**

Die vorliegende Erfindung betrifft ein Verfahren zur Transfektion einer eukaryotischen Zelle, wobei eine Zusammensetzung, die mindestens ein Saccharid und mindestens ein Protein sowie optional ein Salz und/oder eine pH-Wert stabilisierende Komponente aufweist, mit einer Nukleinsäure und einem Transfektionsreagenz in Kontakt gebracht wird, ein Komplex aus Nukleinsäure und Transfektionsreagenz unter Wechselwirkung mit der Zusammensetzung ausgebildet wird sowie der Komplex in eine eukaryotische Zelle transfiziert wird.

SEQUENCE LISTING

<110>  QIAGEN GmbH

<120>  Verfahren zur Transfektion einer eukaryotischen Zelle

<130>  PA537-EP

<160>  15

<170>  PatentIn version 3.3

<210>  1
<211>  22
<212>  DNA
<213>  Artificial

<220>
<223>  Oligonucleotide primer sequence

<400>  1
gcatctcatc ctgaagttgc tt                                                22

<210>  2
<211>  20
<212>  DNA
<213>  Artificial

<220>
<223>  Oligonucleotide primer sequence

<400>  2
caagaaggag ggtgacctga                                                   20

<210>  3
<211>  21
<212>  DNA
<213>  Artificial

<220>
<223>  Oligonucleotide primer sequence

<400>  3
caggattttc agagctttgg g                                                 21

<210>  4
<211>  27
<212>  DNA
<213>  Artificial

<220>
<223>  Oligonucleotide primer sequence

<400>  4
gctggatcat agattaacat tttcgag                                           27

<210>  5
<211>  21
<212>  DNA
<213>  Artificial

<220>
<223>  Oligonucleotide primer sequence

<400>  5

cgacaaagga aatctgagaa a                                                21

<210>  6
<211>  21
<212>  DNA
<213>  Artificial

<220>
<223>  Oligonucleotide primer sequence

<400>  6
gggagatgtt gttgacttgt g                                                21

<210>  7
<211>  20
<212>  DNA
<213>  Artificial

<220>
<223>  Oligonucleotide primer sequence

<400>  7
gcctcaagat catcagcaat                                                  20

<210>  8
<211>  20
<212>  DNA
<213>  Artificial

<220>
<223>  Oligonucleotide primer sequence

<400>  8
gagtccttcc acgataccaa                                                  20

<210>  9
<211>  21
<212>  DNA
<213>  Artificial

<220>
<223>  siRNA

<400>  9
gcgccagaau ggagaugaut t                                                21

<210>  10
<211>  21
<212>  RNA
<213>  Artificial

<220>
<223>  siRNA

<400>  10
uugaguaacg agcugacccc a                                                21

<210>  11
<211>  20
<212>  DNA
<213>  Artificial

<220>

<223>  siRNA

<400>  11
uuuuaaacuc ccuuguugat                                                    20


<210>  12
<211>  21
<212>  DNA
<213>  Artificial

<220>
<223>  siRNA

<400>  12
uuuguuuccu gcaguaguug g                                                  21


<210>  13
<211>  21
<212>  RNA
<213>  Artificial

<220>
<223>  siRNA

<400>  13
uauucauucu ucuugauccg g                                                  21


<210>  14
<211>  21
<212>  DNA
<213>  Artificial

<220>
<223>  siRNA

<400>  14
ggccaagauc caagauaaat t                                                  21


<210>  15
<211>  21
<212>  RNA
<213>  Artificial

<220>
<223>  siRNA

<400>  15
uuuguaaucg ucgauacccu g                                                  21

## Beschreibung

Die vorliegende Erfindung betrifft ein Verfahren zur Transfektion einer eukaryotischen Zelle, wobei eine Zusammensetzung, die mindestens ein Saccharid und mindestens ein Protein sowie optional ein Salz und/oder eine pH-Wert stabilisierende Komponente aufweist, mit einer Nukleinsäure und einem Transfektionsreagenz in Kontakt gebracht wird, ein Komplex aus Nukleinsäure und Transfektionsreagenz unter Wechselwirkung mit der Zusammensetzung ausgebildet wird, sowie der Komplex in eine eukaryotische Zelle transfiziert wird.

Aus dem Stand der Technik sind zahlreiche unterschiedliche Methoden bekannt, um Fremd-Nukleinsäuren, insbesondere Fremd-DNA und Fremd-RNA, in eine Zelle mittels Transfektion einzubringen. Insbesondere werden im Stand der Technik kationische Polymere beschrieben, die mit der zu transfizierenden Nukleinsäure Komplexe eingehen.

Zu diesen kationischen Polymeren zählen beispielsweise Polyethylenimin (PEI) sowie Homopolymere basischer Aminosäuren wie beispielsweise Poly-L-Lysin. Auch dendritische kationische Polymere, wie etwa Polyamidoamine (PAMAMs), sowie Polyamide, Polyallyamine, kationische Methacrylate, Chitosan oder Poly(D,L-Lactid-coGlycolid (PLGAs) sind als geeignete kationische Polymere zur Transfektion von Zellen beschrieben worden.

Des weiteren werden im Stand der Technik Komplexe aus Lipid-basierten Reagenzien und Nukleinsäuren beschrieben, um Fremd-Nukleinsäuren in eine Zelle mittels Transfektion einzubringen. Zu diesen zählen beispielsweise kationische Lipidvesikel, an deren Oberfläche die Nukleinsäure komplexiert wird. Beispiele für Lipid-basierte Reagenzien sind DOTMA (N-[1-(2,3-Dioleyloxy)propyl]-N,N,N-trimethylammomiunchlorid) sowie Mischungen von DOTMA mit Phospholipiden, aus denen positiv geladene Liposomen hergestellt werden, an die die negativ geladene Nukleinsäure bindet.

Auch kommerziell erhältliche Transfektionsreagenzien wie HiPerFect (QIAGEN), Attractene (QIAGEN) und HT-Fect (QIAGEN) bestehen häufig aus Lipid-basierten Reagenzien, mit denen die zu transfizierende Nukleinsäure komplexiert wird.

Üblicherweise sind die Komplexe aus Transfektionsreagenz und zu transfizierender Nukleinsäure nur für eine kurze Zeitspanne, in etwa eine bis wenige Stunden, stabil, so dass diese Komplexe im Allgemeinen erst unmittelbar vor der Transfektion hergestellt werden können. Eine Lagerung dieser Komplexe über einen längeren Zeitraum hinweg oder eine Trocknung (Lyophilisieren) dieser Komplexe ist nicht möglich, ohne dass die Effizienz der nachfolgenden Transfektion stark reduziert wäre, oder dass eine Transfektion überhaupt nicht mehr möglich wäre.

Der vorliegenden Erfindung liegt die Aufgabe zugrunde, die sich aus dem Stand der Technik ergebenden Nachteile zu überwinden und ein Verfahren zur Transfektion einer eukaryotischen Zelle zur Verfügung zu stellen.

Ferner liegt der vorliegenden Erfindung die Aufgabe zugrunde, ein Verfahren zur Transfektion einer eukaryotischen Zelle zur Verfügung zu stellen, bei der der Komplex aus Nukleinsäure und Transfektionsreagenz so stabilisiert wird, dass er einem Lagerungsschritt unterzogen werden kann.

Auch liegt der vorliegenden Erfindung die Aufgabe zugrunde, eine Zusammensetzung zur Verfügung zu stellen, die es ermöglicht, einen Komplex aus einer Nukleinsäure und einem Transfektionsreagenz zu stabilisieren, so dass dieser Komplex lagerbar ist.

Die Aufgabe wird durch die im Anspruchsatz definierten Gegenstände gelöst.

So stellt die vorliegende Erfindung ein Verfahren zur Transfektion einer eukaryotischen Zelle zur Verfügung, aufweisend die folgenden Verfahrensschritte:
a. In Kontakt Bringen einer Zusammensetzung, aufweisend mindestens ein Saccharid und mindestens ein Protein, sowie optional ein Salz und/oder eine pH-Wert stabilisierende Komponente, mit einer Nukleinsäure und einem Transfektionsreagenz;
b. Ausbilden eines Komplexes aus Nukleinsäure und Transfektionsreagenz unter Wechselwirkung mit der Zusammensetzung aus Schritt a;
c. Transfektion einer eukaryotischen Zelle mittels des Komplexes aus Schritt b.

Die vorliegende Erfindung betrifft weiterhin die Verwendung einer Zusammensetzung zur Stabilisierung eines Komplexes aus einer Nukleinsäure und einem Transfektionsreagenz, dadurch gekennzeichnet, dass die Zusammensetzung mindestens ein Saccharid und mindestens ein Protein aufweist, sowie optional ein Salz und/oder eine pH-Wert stabilisierende Komponente.

Die vorliegende Erfindung betrifft des weiteren eine Zusammensetzung zur Lagerung eines Komplexes aus einer Nukleinsäure und einem Transfektionsreagenz, dadurch gekennzeichnet, dass sie mindestens ein Saccharid und mindestens ein Protein sowie eine Nukleinsäure und ein Transfektionsreagenz aufweist, sowie optional ein Salz und/oder eine pH-Wert stabilisierende Komponente.

Völlig überraschend wurde festgestellt, dass sich mit Hilfe dieser Zusammensetzung nicht nur sehr effizient eukaroytische Zellen mit einer Nukleinsäure bei geringer Cytotoxizität transfizieren lassen, sondern dass darüber hinaus diese Zusammensetzung den Komplex aus Nukleinsäure und Transfektionsreagenz so stabilisiert, dass der Komplex aus Nukleinsäure und Transfektionsreagenz lagerbar wird.

Aus diesem Grunde lässt sich im Anschluss an Schritt b es erfindungsgemäßen Verfahrens ein Lagerungsschritt einfügen.

Unter Transfektion wird im Sinne der vorliegenden Erfindung das Einbringen von fremder Nukleinsäure in eine eukaryotische Zelle mit Hilfe eines Transfektionsreagenzes verstanden.

Die eukaroytische Zelle, in die eine Nukleinsäure im erfindungsgemäßen Verfahren eingebracht wird, kann eine Zellkulturzelle sein, es kann sich bei dieser aber auch um eine Zelle oder einen Gewebeverband handeln, der direkt aus einem eukaroytischen Organismus gewonnen wurde.

In einer bevorzugten Ausführungsform handelt es sich bei der eukaryotischen Zelle um eine Zellkulturzelle.

Bei der eukaryotischen Zelle kann es sich um eine menschliche Zelle, eine Zelle aus einem Säugetier, aus einem Vertebraten oder einem Invertebraten handeln. Die eukaryotische Zelle kann aber auch pflanzlichen Ursprungs sein oder von einem Pilz stammen. Bei der eukaryotischen Zelle kann es sich ebenfalls um einen einzelligen Organismus, wie beispielsweise eine Hefe handeln.

Die Nukleinsäure, die im Verfahrensschritt a des erfindungsgemäßen Verfahrens mit einem Transfektionsreagenz und einer erfindungsgemäßen Zusammensetzung in Kontakt gebracht wird, kann eine Ribonukleinsäure (RNA), Desoxyribonukleinsäure (DNA) oder auch eine gemischte Form sein, wobei eine Ribonukleinsäure bevorzugt ist.

Besonders bevorzugt ist eine kurze Ribonukleinsäure wie siRNA, miRNA, piRNA, snRNA oder snoRNA, darüber hinaus besonders bevorzugt handelt es sich bei der kurzen Ribonukleinsäure um eine siRNA.

Unter dem Begriff siRNA wird eine doppelsträngige Ribonukleinsäure verstanden, die eine Länge von etwa 18 bis 30 Nukleotiden aufweist und die in den sogenannten RISC Enzymkomplex (RNA-induced silecing complex) eingebaut werden kann.

Im allgemeinen Fall kann es sich um jeden Typ von Nukleinsäure handeln, die ein N-Glykosid oder C-Glykosid einer Purin- oder Pyrimidinbase oder einer modifizierten Purin- oder Pyrimidinbase darstellt. Die Nukleinsäure kann also auch solche Basen enthalten, die in der Natur nicht vorkommen, beispielsweise Inosin oder Ribo-Thymidin.

Außer der Base selbst kann auch der Zuckerrest modifiziert sein. Eingeschlossen sind Nukleinsäuren mit modifizierter Verknüpfung der Untereinheiten, etwa Phosphorothioat- oder -amidat-Verknüpfungen.

Die Nukleinsäure, die in Schritt a des erfindungsgemäßen Verfahrens mit einem Transfektionsreagenz und einer erfindungsgemäßen Zusammensetzung in Kontakt gebracht wird, kann einzel-, doppel- oder mehrsträngig, linear oder zirkulär sein. Sie kann einem in einer Zelle vorkommenden Molekül entsprechen, wie etwa genomische DNA, Boten-RNA (mRNA) oder miRNA, oder *in vitro* erzeugt werden wie beispielsweise Komplementär-DNA (cDNA), siRNA oder synthetische Nukleinsäuren.

Die Nukleinsäure kann aus wenigen Nukleotiden, bevorzugt acht oder mehr Nukleotiden bestehen, wie etwa Oligonukleotide, siRNA oder miRNA, mehreren hundert Nukleotiden bis hin zu mehreren tausend Nukleotiden, wie sie etwa bestimmte Expressionsvektoren aufweisen.

In einer bevorzugten Ausführungsform enthält die Nukleinsäure keine kodierende Information für ein Polypeptid, sondern ist vielmehr in der Lage, komplementär an eine mRNA, wie sie in der zu transfizierenden Zelle vorkommt, oder an eine von außen in diese Zelle direkt oder indirekt über einen Expressionsvektor hineingebrachte mRNA zu binden und die Translation dieser mRNA in ein Polypeptid zu reduzieren.

Unter Transfektion wird allgemein der Vorgang verstanden, eine Nukleinsäure in eine eukaryotische Zelle einzubringen. Neben Verfahren wie Elektroporation, Calciumphosphat-Präzipitation und Einbringen der Nukleinsäure mit Hilfe einer Partikelkanone sind heute vor allem Verfahren gebräuchlich, bei denen die Nukleinsäure mit Hilfe eines Transfektionsreagenzes in die Zelle eingebracht wird.

Geeignete Transfektionsreagenzien im Sinne der vorliegenden Erfindung sind beispielsweise hoch verzweigte organische Moleküle, so genannte Dendrimere, kationische Polymere wie beispielsweise Polyethylenimin (PEI), Homopolymerere basischer Aminosäuren wie zum Beispiel Poly-L-Lysin, Polyamide, Polyallyamine, kationische Methacrylate, Chitosan oder Poly(D,L-Lactid-coGlycolid (PLGAs).

Eine bevorzugte Gruppe von Transfektionsreagenzien stellen lipid-basierte Transfektionsreagenzien dar. Zu diesen zählen beispielsweise kationische Lipidvesikel, an deren Oberfläche die Nukleinsäure komplexiert wird. Beispiele für Lipid-basierte Reagenzien sind DOTMA (N-[1-(2,3-Dioleyloxy)propyl]-N,N,N-trimethylammomiunchlorid) sowie Mischungen von DOTMA mit Phospholipiden, aus denen positiv geladene Liposomen hergestellt werden, an die die negativ geladene Nukleinsäure bindet.

Auch kommerziell erhältliche Transfektionsreagenzien wie HiPerFect (QIAGEN), Attractene (QIAGEN) und HT-Fect (QIAGEN) bestehen häufig aus Lipid-basierten Reagenzien, mit denen die zu transfizierende Nukleinsäure komplexiert wird und sind ebenfalls bevorzugte Transfektionsreagenzien im Sinne der vorliegenden Erfindung. Dem Fachmann sind viele weitere geeignete Transfektionsreagenzien aus den verschiedenen chemischen Klassen bekannt.

Die Zusammensetzung, mit der die Nukleinsäure und das Transfektionsreagenz in Schritt a des erfindungsgemäßen Verfahrens in Kontakt gebracht werden, weist mindestens ein Saccharid und mindestens ein Protein auf.

Dabei kann es sich beim Saccharid um einen Einfachzucker (Monosaccharid, einen Zweifachzucker (Disaccharid), einen Dreifachzucker (Trisaccharid), einen Vielfachzucker (Polysaccahrid) oder um Mischungen mindestens zweier verschiedener Saccharide handeln. Im Prinzip sind erfindungsgemäß alle Saccharide oder Mischungen von Sacchariden für das erfindungsgemäße Verfahren geeignet, so lange die Saccharide als solche nicht auf 100% der im Verfahren eingesetzten eukaryotischen Zellen toxisch wirken.

Der Fachmann kann durch einfache Vorversuche durch optische Kontrolle der eukaroytischen Zelle feststellen, welche Saccharide in welchem Maße einen toxischen Effekt auf die von ihm verwendete eukaryotische Zelle ausüben, da sich durch cytotoxische Effekte die Morphologie der eukaryotischen Zelle verändert.

Beispiele für geeignete Monosaccharide sind Glucose, Mannose, Fructose, Ribose, Desoxyribose, Galactose, Fucose oder Rhamnose. Dem Fachmann sind weitere geeignete Monosaccharide geläufig.

Beispiele für geeignete Disaccharide sind Saccharose, Lactose, Lactulose, Maltose oder Trehalose. Dem Fachmann sind weitere geeignete Disaccharide geläufig.

Beispiele für geeignete Trisaccharide sind Melezitose, Raffinose oder Umbelliferose. Dem Fachmann sind weitere geeignete Trisaccharide geläufig.

Als Polysaacharid kann beispielsweise Stärke eingesetzt werden. Weitere geeignete Polysaccharide sind dem Fachmann geläufig.

In einer bevorzugten Ausführungsform handelt es sich beim Saccharid oder beim Gemisch von Sacchariden um Disaccharide.

In einer besonders bevorzugten Ausführungsform handelt es sich beim Disaccharid um Saccharose oder um Trehalose oder um ein Gemisch von beiden.

In einer ganz besonders bevorzugten Ausführungsform handelt es sich beim Disaccharid um Saccharose.

Die Konzentration des Saccharides kann in jedem Bereich bis zu seiner Löslichkeitsgrenze im verwendeten Puffer liegen, wobei durch die verwendete Konzentration nicht alle Zellen durch cytotoxische Effekte abgetötet werden dürfen.

Der Fachmann kann durch einfache Vorversuche mittels optischer Kontrolle der Morphologie der eukaryotischen Zelle feststellen, ob die verwendete Konzentration des von ihm verwendeten Saccharids eine Änderung in der Morphologie der Zelle hervorruft.

In einer bevorzugten Ausführungsform ist die Konzentration des Saccharids oder des Gemisches von Sacchariden so gewählt, dass mehr als die Hälfte aller eukaryotischen Zellen keine durch Cytotoxizität hervorgerufene Änderung in ihrer Morphologie aufweist.

In einer weiteren bevorzugten Ausführungsform liegt das Saccharid oder das Gemisch von Sacchariden in einer Konzentration von 0,01 M bis 2 M vor, wobei die durch Cytotoxizität hervorgerufene Änderung der Morphologie nicht mehr als die Hälfte aller eukaryotischen Zellen betrifft.

In einer darüber hinaus bevorzugten Ausführungsform liegen die Saccharose oder die Trehalose oder das Gemisch von beiden in einer Konzentration von 0,01 M bis 2 M, darüber hinaus bevorzugt in einer Konzentration von 0,05 M bis 1 M, darüber hinaus bevorzugt in einer Konzentration von 0,1 bis 0,5 M, darüber hinaus bevorzugt in einer Konzentration von 0,1 M vor.

In einer darüber hinaus ganz besonders bevorzugten Ausführungsform liegt die Sacchaorse in einer Konzentration von 0,01 M bis 2 M, darüber hinaus bevorzugt in einer Konzentration von 0,05 M bis 1 M, darüber hinaus bevorzugt in einer Konzentration von 0,1 bis 0,5 M, darüber hinaus ganz besonders bevorzugt in einer Konzentration von 0,1 M vor.

Die Zusammensetzung, mit der die Nukleinsäure und das Transfektionsreagenz in Schritt a des erfindungsgemäßen Verfahrens in Kontakt gebracht werden, weist mindestens ein Saccharid und mindestens ein Protein auf.

Dabei kann es sich beim Protein prinzipiell um ein Protein aus einer oder mehreren Untereinheiten oder um ein Polypeptid aus mehreren Aminosäuren handeln. Es kann sich aber auch um eine Mischung aus zwei oder mehreren Proteinen und/oder Polypeptiden handeln. Das Protein oder die Mischung muss dabei so gewählt sein, dass es aufgrund seiner Ladung die Wechselwirkung zwischen der Nukleinsäure und dem Transfektionsreagenz nicht verhindert, so dass Nukleinsäure und Transfektionsreagenz einen Komplex ausbilden können.

Des weiteren müssen das Protein oder die Mischung so gewählt sein, dass sie in der eukaryotischen Zelle keinen Effekt auslösen, der die Zelle als Reaktion auf dieses Protein veranlasst, bestimmte zelluläre Prozesse hinauf- oder herunter zu regulieren. Das Protein oder die Mischung müssen vielmehr so gewählt sein, dass die zellulären Prozesse der Zelle weitgehend unbeeinflusst bleiben.

Geeignet für das erfindungsgemäße Verfahren sind daher Proteine wie beispielsweise BSA oder andere Albumine oder Mischungen wie fötales Kälberserum.

In einer bevorzugten Ausführungsform handelt es sich beim Protein um BSA.

Die Konzentration des eingesetzten Proteins sollte in einem Bereich liegen, in dem sie in der erfindungsgemäßen Zusammensetzung löslich ist und nicht ausfällt und keine cytotoxischen Effekte auslöst.

In einer bevorzugten Ausführungsform weist das Protein in der erfindungsgemäßen Zusammensetzung einen Gewichtsanteil von 0,1% bis 5% auf, in einer ganz besonders bevorzugten Ausführungsform weist es einen Gewichtsanteil von 0,5% bis 2% auf, darüber hinaus besonders bevorzugt einen Gewichtsanteil von 1 % auf.

In einer besonders bevorzugten Ausführungsform ist das Protein in der erfindungsgemäßen Zusammensetzung BSA mit einem Gewichtsanteil von 0,1% bis 5%, darüber hinaus bevorzugt mit einem Gewichtsanteil von 0,5% bis 2%, darüber hinaus besonders bevorzugt mit einem Gewichtsanteil von 1%.

Zu der erfindungsgemäßen Zusammensetzung kann optional ein Salz hinzugefügt werden. Das Salz kann dazu dienen, das Ausbilden des Komplexes zwischen Nukleinsäure und Transfektionsreagenz zu fördern oder zu stabilisieren oder auch, um die Wechselwirkung zwischen der erfindungsgemäßen Zusammensetzung mit dem Komplex aus Nukleinsäure und Transfektionsreagenz zu fördern oder zu stabilisieren, beispielsweise, indem der Komplex durch Ladungsausgleich stabilisiert wird.

Eine weitere Funktion des Salzes kann es auch sein, das Reaktionsmilieu allgemein zu stabilisieren oder zu einer höheren Effizienz der Transfektion beizutragen.

Beispiele für geeignete Salze sind Halogenide, Phosphate, Nitrate oder Sulfate von Alkali- oder Erdalkalimetallen oder Ammonium-Ionen. Geeignet sind aber auch die Salze von organischen Säuren wie Acetate oder Citrate.

Dem Fachmann sind viele weitere geeignete Salze geläufig, die in der Lage sind wie oben beschrieben das Ausbilden des Komplexes zu fördern oder zu stabilisieren oder allgemein das Reaktionsmilieu zu stabiliseren oder zu einer höheren Effizienz der Transfektion beizutragen. Bei der im erfindungsgemäßen Verfahren verwendeten Zusammensetzung kann optional eine pH-Wert stabilisierende Komponente hinzugefügt werden. Diese Komponente kann durch die Stabilisierung des pH-Wertes dazu dienen, das Ausbilden des Komplexes zwischen Nukleinsäure und Transfektionsreagenz zu fördern oder zu stabilisieren oder auch, um die Wechselwirkung zwischen der erfindungsgemäßen Zusammensetzung mit dem Komplex aus Nukleinsäure und Transfektionsreagenz zu fördern oder zu stabilisieren.

Eine weitere Funktion der pH-Wert stabilisierenden Komponente kann es auch sein, den pH-Wert für die nachfolgende Transfektion in einen geeigneten Bereich zu brigen.

Beispiele für geeignete pH-Wert stabilisierende Komponenten sind HBS, PBS und Optimem (Life Technologies).

Nachdem in Schritt a des erfindungsgemäßen Verfahrens die Zusammensetzung, aufweisend mindestens ein Saccharid und mindestens ein Protein, sowie optional ein Salz und/oder eine pH-Wert stabilisierende Komponente, mit einer Nukleinsäure und einem Transfektionsreagenz in Kontakt gebracht worden ist, bildet sich in Schritt b des erfindungsgemäßen Verfahrens ein Komplex aus Nukleinsäure und Transfektionsreagenz aus. Dieser Komplex beruht auf nicht-kovalenten Wechselwirkungen, in der Hauptsache auf ionischen Wechselwirkungen, zwischen der Nukleinsäure und dem Transfektionsreagenz. Dieser Komplex aus Transfektionsreagenz und Nukleinsäure wechselwirkt nicht-kovalent mit der Zusammensetzung aus Schritt a des erfindungsgemäßen Verfahrens, wobei diese Wechselwirkung stabilisierend wirkt.

In Schritt c des erfindungsgemäßen Verfahrens wird eine eukaryotische Zelle mittels des Komplexes aus Schritt b des erfindungsgemäßen Verfahrens transfiziert.

Völlig überraschend wurde festgestellt, dass die erfindungsgemäße Zusammensetzung es ermöglicht, den Komplex aus Nukleinsäure und Transfektionsreagenz lagerungsfähig zu machen, und es nach der Lagerung möglich ist, eine eukaryotische Zelle mit diesem Komplex zu transfizieren.

Aus diesem Grunde ist es möglich, nach dem Ausbilden eines Komplexes aus Nukleinsäure und Transfektionsreagenz unter Wechselwirkung mit der Zusammensetzung aus Schritt a des erfindungsgemäßen Verfahrens, den Komplex nach Schritt b zu lagern, bevor eine eukaryotische Zelle mittels des Komplexes aus Schritt b in Schritt c transfiziert wird.

Die Lagerung des Komplexes kann dabei so durchgeführt werden, dass der Komplex zur Lagerung getrocknet wird. Hierzu wird dem Komplex aus Nukleinsäure und Transfektionsreagenz, der mit der erfindungsgemäßen Zusammensetzung wechselwirkt, die Flüssigkeit entzogen.

Das Entziehen der Flüssigkeit kann dadurch geschehen, dass der Komplex mit der Zusammensetzung im Vakuum getrocknet wird. Dies kann zum Beispiel mittels einer Vakuumglocke erfolgen, oder aber auch mit Hilfe einer Vakuumzentrifugation, oder mittels einer Gefriertrocknungsanlage.

Die Trocknung kann aber auch bei normalem Atmosphärendruck stattfinden. Hierzu kann der Komplex mit der erfindungsgemäßen Zusammensetzung bei Raumtemperatur in einem offenen Behältnis stehen gelassen werden, bis die Flüssigkeit verdunstet ist. Es ist auch möglich, den Verdunstungsvorgang zu beschleunigen, indem das Behältnis mit dem Komplex und der erfindungsgemäßen Zusammensetzung auf Temperaturen oberhalb der Raumtemperatur erhitzt wird. Dabei muss die Temperatur jedoch in einem Bereich verbleiben, in dem weder die Nukleinsäure noch das Transfektionsreagenz thermisch zerstört oder irreversibel denaturiert werden. Dadurch ist es möglich, innerhalb sehr kurzer Zeit und mit sehr geringem technischen Aufwand, Komplexe aus Transfektionsreagenz und Nukleinsäure zu generieren, die sich über einen längeren Zeitraum hinweg lagern lassen und die nach der Lagerung erfolgreich in eine eukaryotische Zelle transfiziert werden können. Wenn der Komplex zur Lagerung getrocknet wird, ist es erforderlich, dass vor der Transfektion einer eukaroytischen Zelle der Komplex mit der erfindungsgemäßen Zusammensetzung wieder in einer Flüssigkeit aufgenommen wird.

Bei dieser Flüssigkeit kann es sich um Wasser handeln, es kann sich dabei aber auch um einen Puffer handeln, der Salze und/oder eine pH-Wert stabilisierende Komponente enthält. Schließlich kann es sich bei dieser Flüssigkeit auch um eine Komponente handeln, die die Transfektionseffizienz erhöht. Dabei kann es sich beispielsweise um ein organisches Molekül handeln, das auf das jeweilige Transfektionsreagenz abgestimmt ist und im Zusammenspiel mit dem Transfektionsreagenz die Transfektionseffizienz erhöht. Dieses Molekül wird auch als Enhancer bezeichnet.

Die Lagerung kann, unabhängig davon, ob der Komplex mit der erfindungsgemäßen Zusammensetzung zur Lagerung getrocknet wird oder in flüssigem Zustand verbleibt, bei Raumtemperatur (20 °C bis 25 °C) erfolgen. Dies hat den großen Vorteil, dass es für das erfindungsgemäße Verfahren nicht erforderlich ist, den Komplex mit der erfindungsgemäßen Zusammensetzung in einem Kühlschrank, einem Kühlraum oder einer Gefriertruhe lagern zu müssen, so dass wertvoller Raum für empfindlichere Substanzen genutzt werden kann. Auch ist es so leicht möglich, den Komplex mit der erfindungsgemäßen Zusammensetzung bei Raumtemperatur zu versenden. Dies spart Kosten ein, da auf einen Versand in einer Kühlbox verzichtet werden kann. Außerdem ist dadurch gewährleistet, dass eine Verzögerung im Versand, bei dem die Proben auftauen, der Komplex aus Transfektionsreagenz und Nukleinsäure immer noch sehr gut für die Transfektion einer eukaryotischen Zelle geeignet ist.

Es ist aber auch möglich, die Lagerung bei einer Temperatur durchzuführen, die unterhalb der Raumtemperatur liegt.

So ist es beispielsweise möglich, die Lagerung bei Temperaturen von 2 °C bis 8 °C im Kühlschrank oder einer Kühlkammer durchzuführen.

Ebenfalls ist es beispielsweise möglich, die Lagerung bei Temperaturen unter 0 °C durchzuführen, beispielsweise durch Kühlen auf -20 °C oder Temperaturen, die noch unterhalb dieser Temperatur liegen.

Liegen die Temperaturen für die Lagerung unterhalb des Gefrierpunktes der Flüssigkeit, in der sich der Komplex und die erfindungsgemäße Zusammensetzung befinden, so muss die Flüssigkeit vor der Transfektion einer eukaryotischen Zelle wieder als Flüssigkeit vorliegen.

Auch für die Lagerung bei Temperaturen unterhalb von Raumtemperatur ist es nicht wesentlich, ob der Komplex mit der erfindungsgemäßen Zusammensetzung getrocknet wird oder im flüssigen Zustand verbleibt.

Die Lagerung des Komplexes mit der erfindungsgemäßen Zusammensetzung kann dabei für einen kurzen Zeitraum erfolgen, beispielsweise für mehrere Stunden oder einige Tage, es ist aber auch möglich, den Komplex über mehrere Wochen oder Monate hinweg zu lagern. Dabei ist die mögliche Lagerungszeit umso länger, je niedriger die Lagerungstemperatur gewählt ist.

In Schritt c des erfindungsgemäßen Verfahrens die Transfektion einer eukaryotischen Zelle durchgeführt. Dabei ist es möglich, den Komplex mit der erfindungsgemäßen Zusammensetzung direkt mit den zu transfizierenden Zellen zusammenzugeben, sofern der Komplex nicht getrocknet wurde und die Lagerung oberhalb des Gefrierpunktes des Komplexes stattfand.

Fand die Lagerung unterhalb des Gefrierpunktes statt, kann direkt nach dem Auftauen die Transfektion durchgeführt werden, da der Komplex aus Transfektionsreagenz und Nukleinsäure durch die erfindungsgemäße Zusammensetzung so stabilisiert wurde, dass eine erfolgreiche Transfektion ohne weiteren Behandlungsschritt möglich ist.

Fand die Lagerung in getrocknetem Zustand stand, kann die Transfektion direkt nach Aufnahme des Komplexes mit der erfindungsgemäßen Zusammensetzung in Wasser oder in einer anderen geeigneten Flüssigkeit erfolgen, da der Komplex aus Transfektionsreagenz und Nukleinsäure durch die erfindungsgemäße Zusammensetzung so stabilisiert wurde, dass eine erfolgreiche Transfektion ohne weiteren Behandlungsschritt möglich ist.

Es ist aber auch möglich, dass vor der Transfektion dem Komplex mit der erfindungsgemäßen Zusammensetzung noch weitere Komponenten hinzugefügt werden, beispielsweise Salze, pH-Wert stabilisierende Komponenten oder Enhancer des Transfektionsreagenzes.

Dem Fachmann sind solche geeigneten Komponenten und Enhancer geläufig.

In einer bevorzugten Ausführungsform werden dem Komplex mit der erfindungsgemäßen Zusammensetzung vor der Transfektion einer eukaryotischen Zelle keine weiteren Komponenten hinzugefügt, da die erfindungsgemäße Zusammensetzung zusammen mit dem Komplex aus Nukleinsäure und Transfektionsreagenz alle für eine effiziente Transfektion erforderlichen Bestandteile erhält und so auf weitere Behandlungsschritte verzichtet werden kann.

War der Komplex mit der erfindungsgemäßen Zusammensetzung in getrocknetem Zustand gelagert worden, so wird dieser in einer bevorzugten Ausführungsform in Wasser aufgenommen.

Abbildungen:

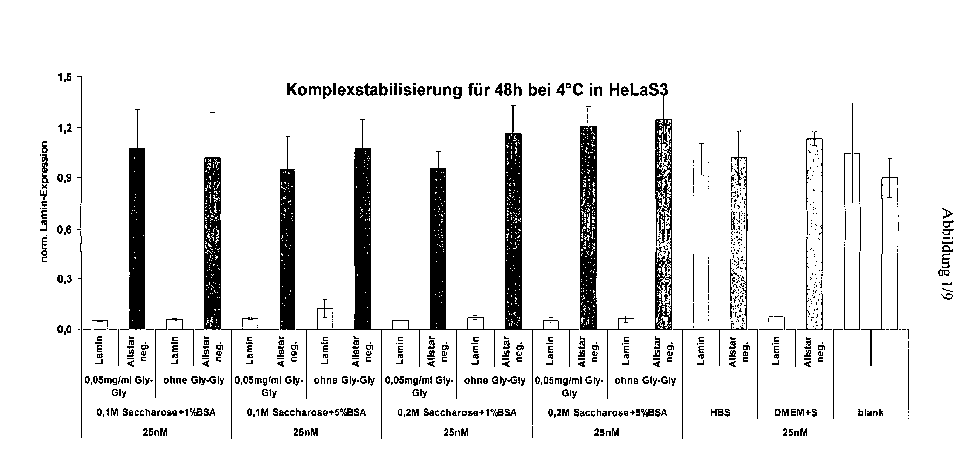

Abbildung 1 zeigt die Analyse eines Gen-Silencing Effektes mittels qPCR nach der Transfektion von HeLa S3 Zellen mit einer gegen Lamin gerichteten siRNA nach 2 Tagen Lagerung bei 4 °C. Vor der Lagerung wurden unterschiedliche Zusammensetzungen zu der siRNA und dem Transfektionsreagenz gegeben.

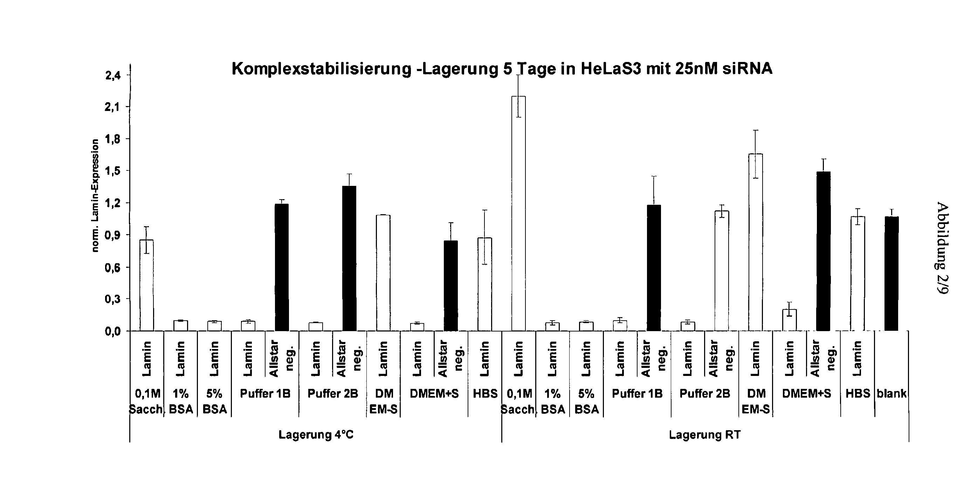

Abbildung 2 zeigt die Analyse eines Gen-Silencing Effektes mittels qPCR nach der Transfektion von HeLa S3 Zellen mit einer gegen Lamin gerichteten siRNA nach 5 Tagen Lagerung bei 4 °C oder Raumtemperatur. Vor der Lagerung wurden unterschiedliche Zusammensetzungen zu der siRNA und dem Transfektionsreagenz gegeben.

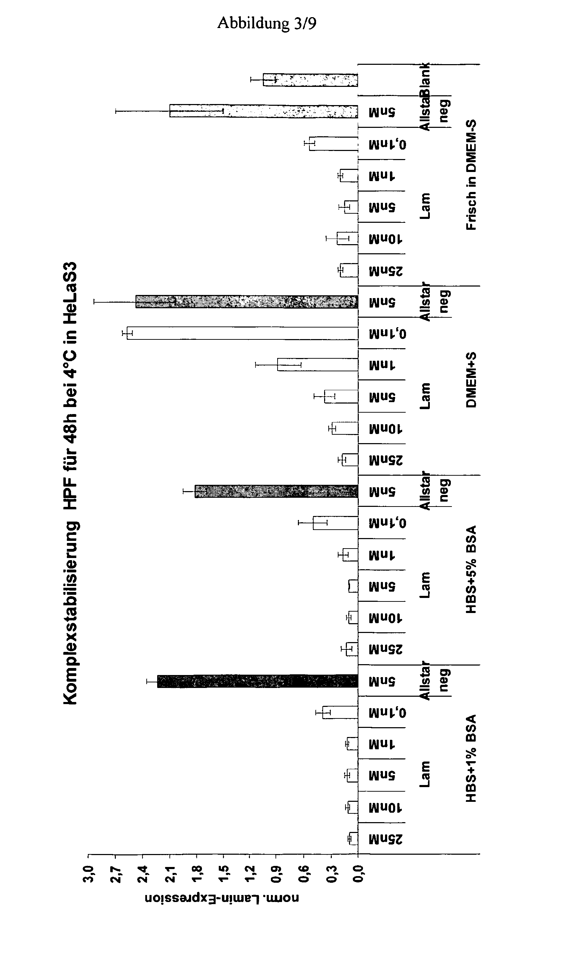

Abbildung 3 zeigt die Analyse eines Gen-Silencing Effektes mittels qPCR nach der Transfektion von HeLa S3 Zellen mit einer gegen Lamin gerichteten siRNA in unterschiedlichen Konzentrationen nach 2 Tagen Lagerung bei 4 °C. Vor der Lagerung wurden unterschiedliche Zusammensetzungen zu der siRNA und dem Transfektionsreagenz gegeben. Zum Vergleich wurden frisch angesetzte Transfektionskomplexe transfiziert.

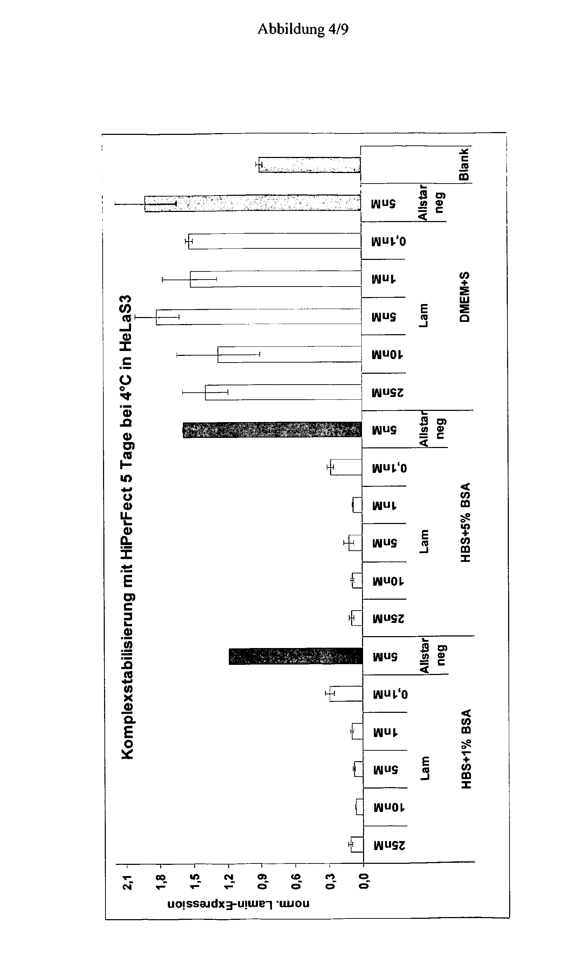

Abbildung 4 zeigt die Analyse eines Gen-Silencing Effektes mittels qPCR nach der Transfektion von HeLa S3 Zellen mit einer gegen Lamin gerichteten siRNA in unterschiedlichen Konzentrationen nach 5 Tagen Lagerung bei 4 °C. Vor der Lagerung wurden unterschiedliche Zusammensetzungen zu der siRNA und dem Transfektionsreagenz gegeben.

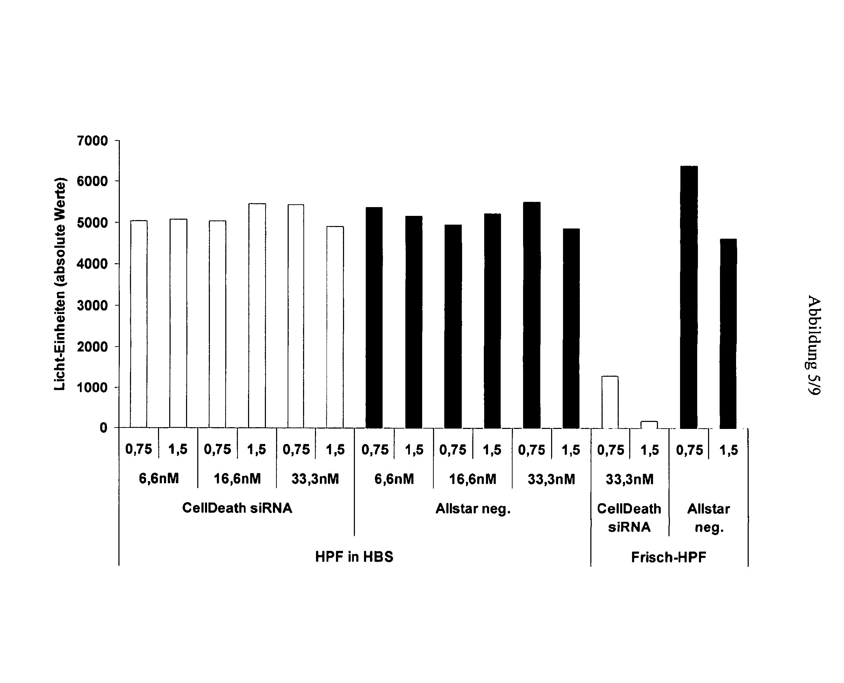

Abbildung 5 zeigt die Analyse der Überlebensrate von HeLa S3 Zellen, die mit einer siRNA, die Zelltod auslöst, transfiziert worden waren. Transfiziert wurden getrocknete Komplexe von Transfektionsreagenz mit siRNA im Vergleich zu frisch angesetzten Komplexen. In keinem Fall wurde die erfindungsgemäße Zusammensetzung zum Komplex gegeben.

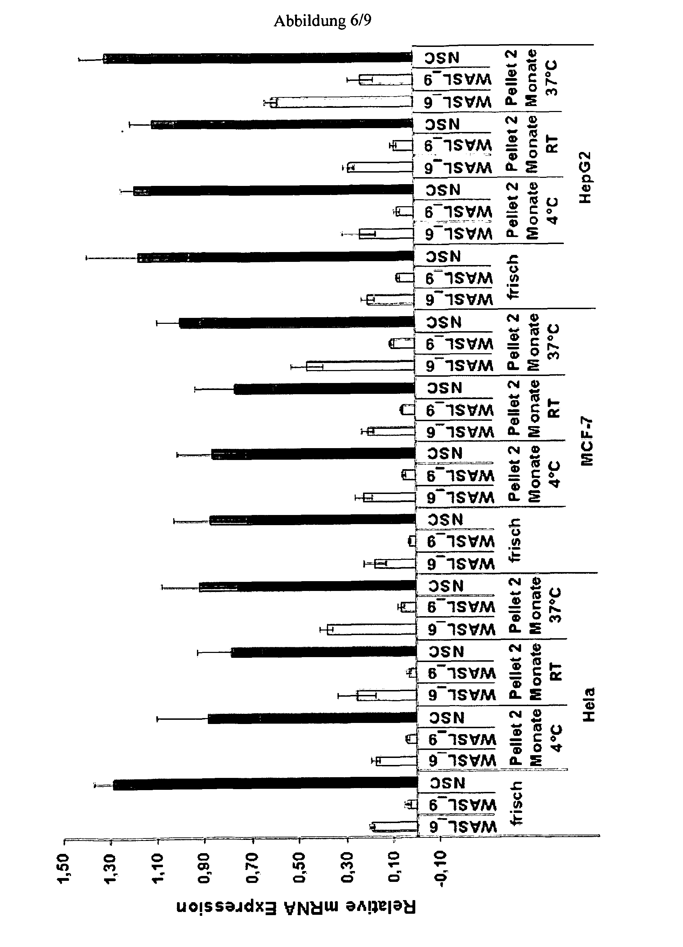

Abbildung 6 zeigt die Analyse eines Gen-Silencing Effektes mittels qPCR nach der Transfektion von HeLa S3, MCF-7 bzw. HepG2 Zellen mit zwei verschiedenen siRNAs (WASL_6, WASL_9), die gegen WASL gerichtet sind. Die Transfektionskomplexe wurden zusammen mit der erfindungsgemäßen Zusammensetzung getrocknet und für 2 Monate bei 4 °C, Raumtemperatur bzw. 37 °C gelagert.

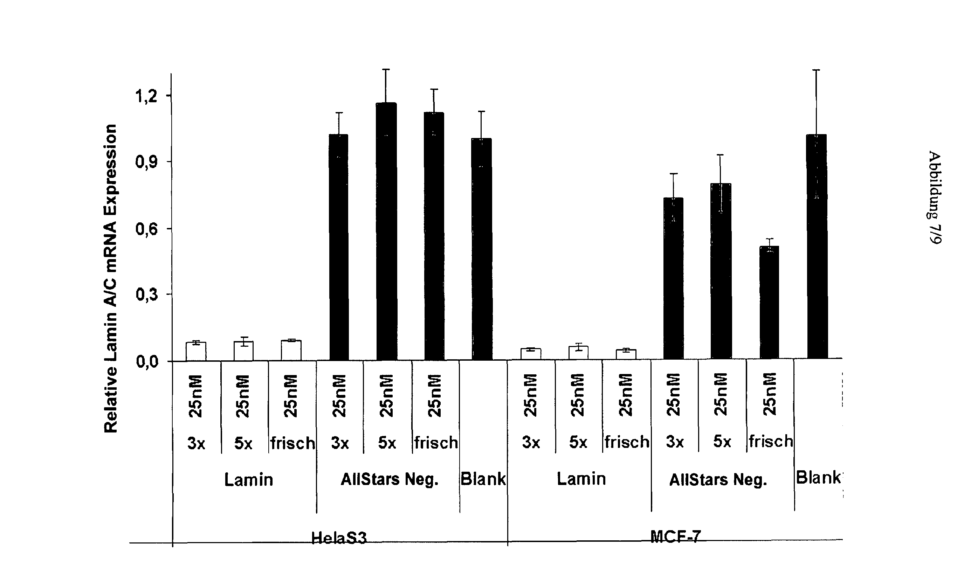

Abbildung 7 zeigt die Analyse eines Gen-Silencing Effektes mittels qPCR nach der Transfektion von HeLa S3 und MCF-7 Zellen mit einer gegen Lamin gerichteten siRNA. Zum Transfektionskomplex aus siRNA und Transfektionsreagenz wurde die erfindungsgemäße Zusammensetzung gegeben und 3 oder 5 Einfrier- und Auftauzyklen unterzogen, bevor die Zellen transfiziert wurden. Zur Kontrolle wurden frische Transfektionskomplexe transfiziert.

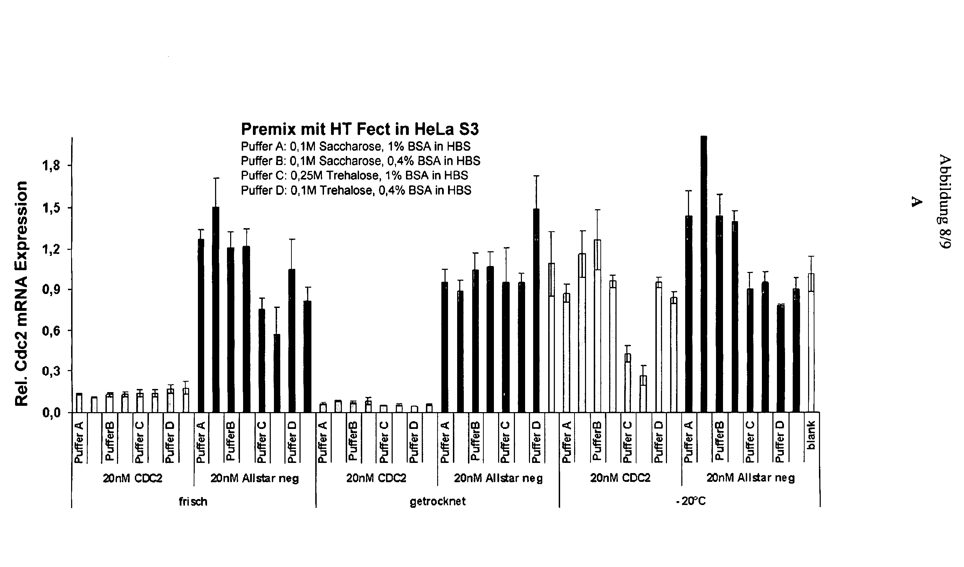

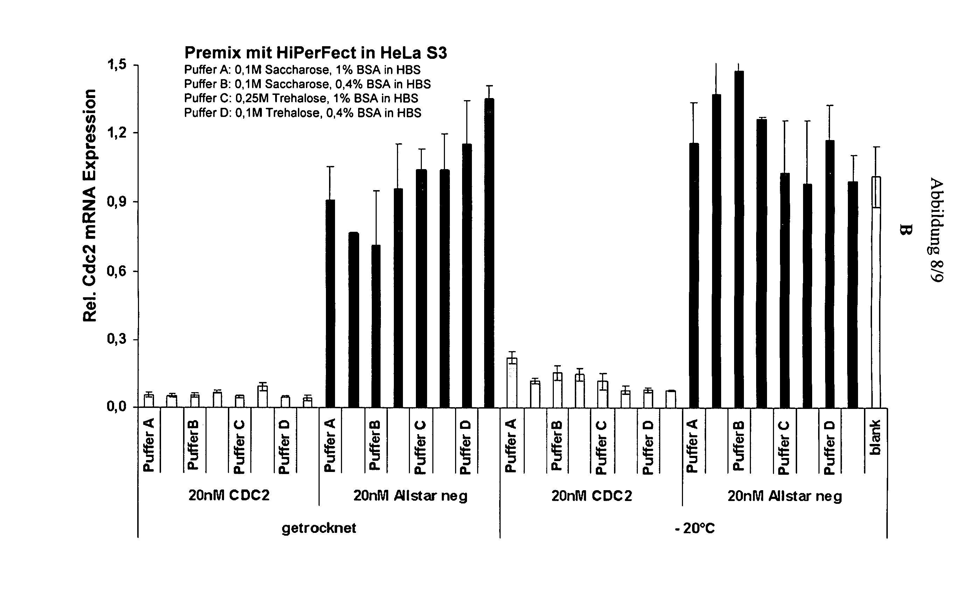

Abbildung 8 zeigt die Analyse eines Gen-Silencing Effektes mittels qPCR nach der Transfektion von HeLa S3 Zellen mit einer siRNA, die gegen CDC2 gerichtet ist. Der Transfektionskomplex wurde mit HT-Fect (QIAGEN) bzw. HiPerFect (QIAGEN) als Transfektionsreagenz gebildet. Zum Transfektionskomplex aus siRNA und Transfektionsreagenz wurden unterschiedliche Zusammensetzungen gegeben. Ein Teil der Komplexe mit Zusammensetzung wurde frisch transfiziert, ein weiterer Teil getrocknet und wieder ein Wasser aufgenommen, ein dritter Teil wurde bei -20 °C gelagert.

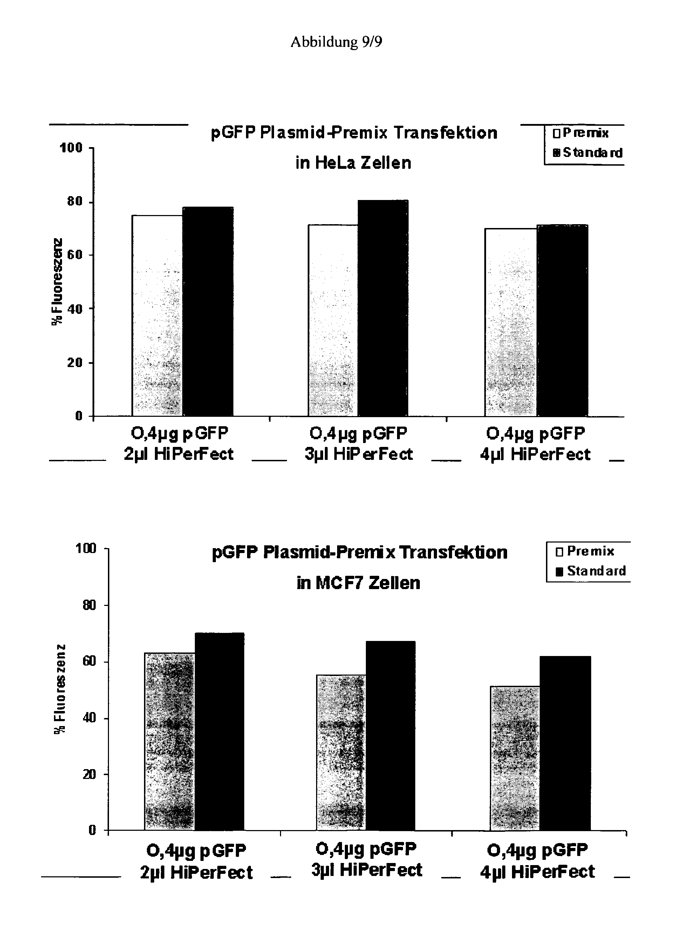

Abbildung 9 zeigt die quantitative Bestimmung der GFP-positiven Zellen in % aller gemessenen Zellen nach der Transfektion von HeLa S3 und MCF-7 Zellen mit einem Plasmid, das für ein Green Fluorescent Protein (GFP) kodiert. Der Komplex aus Plasmid und Transfektionsreagenz wurde zusammen mit der erfindungsgemäßen Zusammensetzung im Vakuum getrocknet und bei 4 °C gelagert.

### Beispiele:

Die nachfolgenden Beispiele sind dazu gedacht, die Erfindung weiter zu erläutern, ohne dass diese auf die Ausführungsbeispiele beschränkt sein soll.

### Verwendete Sequenzen:

| Primer: | |
|---|---|
| 3Lamin3815: | 5'-GCATCTCATCCTGAAGTTGCTT |
| 3Lamin8: | 5'-CAAGAAGGAGGGTGACCTGA |
| CDC2F: | 5'-CAGGATTTTCAGAGCTTTGGG |
| CDC2R: | 5'-GCTGGATCATAGATTAACATTTTCGAG |
| WASLF: | 5'-CGACAAAGGAAATCTGAGAAA |
| WASLR: | 5'-GGGAGATGTTGTTGACTTGTG |
| GAPDHF: | 5'-GCCTCAAGATCATCAGCAAT |
| GAPDHR: | 5'-GAGTCCTTCCACGATACCAA |

### siRNAs (Guide-Strang):

| | |
|---|---|
| Lamin A/C: | 5'-rGrCrGrCrCrArGrArArUrGrGrArGrArUrGrArUTT |
| Cdc2_641: | 5'-rUrUrGrArGrUrArArCrGrArGrCrUrGrArCrCrCrCrA |
| WASL_6: | 5'-rUrUrUrUrArArArCrUrCrCrCrUrUrGrUrUrGAT |
| WASL_9: | 5'-rUrUrUrGrUrUrUrCrCrUrGrCrArGrUrArGrUrUGG |
| Hs_plk1: | 5'-rUrArUrUrCrArUrUrCrUrUrCrUrUrGrArUrCrCrGrG |
| Hs_Ubb: | 5'-rGrGrCrCrArArGrArUrCrCrArArGrArUrArArATT |
| NSC: | 5'-rUrUrUrGrUrArArUrCrGrUrCrGrArUrArCrCrCrUrG |

### Beispiel 1:

Stabilisierung eines Komplexes von Transfektionsreagenz und siRNA mit verschiedenen Zusammensetzungen bei einer Lagerung des Komplexes für 2 Tage bei 4 °C

In einem Gesamtvolumen von 100 µl Puffer HBS (20 mM Hepes, 150 mM NaCl, pH 7,4) wurden 20 nM Lamin A/C bzw. 20 nM Non Silencing Control (NSC, Allstar neg.) siRNA hinzugegeben, sowie 3 µl des Transfektionsreagenzes HiPerfect (QIAGEN) sowie unterschiedliche Stabilisatoren des Komplexes aus Transfektionsreagenz und Nukleinsäure.

Diese waren (jeweils finale Konzentration):
0,1 M Saccharose, 1% (w/v) BSA
0,1 M Saccharose, 1% (w/v) BSA, 0,05 mg/ml Gly-Gly
0,1 M Saccharose, 5% (w/v) BSA
0,1 M Saccharose, 5% (w/v) BSA, 0,05 mg/ml Gly-Gly
0,2 M Saccharose, 1% (w/v) BSA
0,2 M Saccharose, 1% (w/v) BSA, 0,05 mg/ml Gly-Gly
0,2 M Saccharose, 5% (w/v) BSA
0,2 M Saccharose, 5% (w/v) BSA, 0,05 mg/ml Gly-Gly
DMEM (Invitrogen) + Serum

Die Komplexe mit den Stabilisatoren wurden für 5 bis 10 Minuten bei Raumtemperatur inkubiert und anschließend für 48 Stunden bei 4 °C gelagert.

Nach der Lagerung wurden die Ansätze tropfenweise zu jeweils zu 60.000 HeLa S3 Zellen gegeben und nach dem HiPerFect-fastforward Protokoll (QIAGEN) transfiziert.

Nach einer Inkubation der Zellen für 48 Stunden bei 37 °C in 5% CO₂ wurde die RNA mit Hilfe des RNeasy Protokolls (QIAGEN) isoliert.

Durch die transfizierte Lamin A/C siRNA wird in der erfolgreich transfizierten Zelle die Expression der Lamin mRNA reduziert. Zur Bestimmung der Transfektionseffizienz wurde die Reduktion Expression der Lamin mRNA in Relation zur Experssion der GAPDH mRNA, deren Expression durch die Transfektion unbeeinflusst bleibt, mit Hilfe einer quantitativen RT-PCR bestimmt. Dazu wurden 2 µl einer 1:20 Verdünnung der isolierten RNA nach dem QuantiFast SYBR Green One-Step RT-PCR Protokoll (Qiagen) mit Hilfe der Primer 3Lamin3815 und 3Lamin8 bzw. GAPDHF und GAPDHR in einem 10 µl Ansatz revers transkribiert und anschließend amplifiziert. Die reverse Transkription und die Amplifikation fanden in einem ABI 7900 Cycler (Applied Biosystems) statt, die Reaktionsbedigungen waren:

| | | |
|---|---|---|
| 10 min 50°C | | |
| 5 min 95 °C | | |
| | Danach 40 Zyklen | |
| | | 10 s 95°C |
| | | 30 s 60°C |

Die auf GAPDH normalisierte Expression von Lamin ist in Abbildung 1 gezeigt. Die hellen Balken zeigen die Expression der Lamin mRNA in Zellen, die mit Lamin A/C siRNA transfiziert worden waren, die dunklen Balken zeigen die Expression der Lamin mRNA in Zellen, die mit NSC siRNA transfiziert worden waren, in der Abbildung mit Allstar neg. abgekürzt.

Unter den Balken sind die jeweiligen Zusammensetzungen des Stabilisators angegeben, in HBS wurden keine stabilisierenden Komponenten hinzugegeben, mit "blank" ist die Nullkontrolle bezeichnet.

Abbildung 1 zeigt, dass sich nach einer Lagerung für 2 Tage bei 4 °C der siRNA mit dem Transfektionsreagenz HiPerFect keine HeLa S3 Zellen mehr erfolgreich transfizieren lassen. Die normierte Laminexpression liegt im Bereich der Expression der Non Silencing Control (Allstar neg.). Wurden dem Komplex aus Transfektionsreagenz und siRNA hingegen stabilisierende Zusammensetzungen hinzugegeben, so war nach der Lagerung eine erfolgreiche Transfektion der Lamin siRNA möglich. Dabei zeigte sich, dass die Kombination aus Saccharose und BSA bei allen eingesetzten Konzentrationen denselben stabilisierenden Effekt auf den Komplex aus Transfektionsreagenz und siRNA ausübte. Die Zugabe von Gly-Gly hatte weder einen positiven noch einen negativen Einfluss auf die Stabilisierung. Auch DMEM mit Serum, dessen Proteinanteil zu etwa 90% aus BSA besteht, zeigte sich in diesem Experiment geeignet, um den Komplex zu stabilisieren.

Dieser Versuch hat gezeigt, dass es möglich ist, einen Komplex aus Transfektionsreagenz und siRNA für 2 Tage bei 4 °C zu lagern, ohne dass die Transfektionseffizienz reduziert wird, wenn zu diesem Komplex ein Gemisch aus Saccharose und BSA hinzugegeben wird. Fehlen diese beiden Komponenten, ist eine Transfektion nach der Lagerung nicht mehr möglich.

### Beispiel 2:

Stabilisierung eines Komplexes von Transfektionsreagenz und siRNA mit verschiedenen Zusammensetzungen bei einer Lagerung des Komplexes für 5 Tage bei 4 °C und bei Raumtemperatur

Vorgegangen wurde wie in Beispiel 1 beschrieben, nur dass diesmal der Komplex aus siRNA und Transfektionsreagenz für 5 Tage bei 4 °C bzw. bei Raumtemperatur gelagert wurde. Folgende Zusammensetzungen wurden als Stabilisatoren des Komplexes aus siRNA und Transfektionsreagenz verwendet:
0,1 M Saccharose
1% (w/v) BSA
5% (w/v) BSA
Puffer 1B: 0,1 M Saccharose, 1% (w/v) BSA
Puffer 2B: 0,1 M Saccharose, 5% (w/v) BSA
DMEM ohne Serum
DMEM mit Serum

Das Ergebnis dieses Versuches ist in Abbildung 2 wiedergegeben. Abbildung 2 zeigt, dass es möglich ist, den Komplex aus siRNA und Transfektionsreagenz für 5 Tage so zu stabilisieren, dass es möglich ist, diesen Komplex nach der Lagerung zu transfizieren. Dabei spielt es keine Rolle, ob die Komplexe bei 4 °C oder bei Raumtemperatur gelagert worden sind, die Transfektionseffizienz ist vergleichbar.

Neben einem Gemisch aus Saccharose und BSA ist unter diesen Versuchsbedingungen auch BSA alleine in der Lage, den Komplex aus siRNA und Transfektionsreagenz zu stabilisieren. Auch DMEM mit Serum eignet sich dafür, nicht aber Saccharose alleine oder DMEM ohne Serum.

### Beispiel 3:

Stabilisierung eines Komplexes von Transfektionsreagenz und siRNA mit verschiedenen Zusammensetzungen bei einer Lagerung des Komplexes für 2 Tage bei 4 °C mit unterschiedlichen Konzentrationen an eingesetzter siRNA

Vorgegangen wurde wie in Beispiel 1 beschrieben, nur dass diesmal zusätzlich zu 25 nM siRNA auch geringere Mengen siRNA (10 nM, 5 nM, 1 nM, 0,1 nM) verwendet wurden. Die Lagerung erfolgte diesmal für 2 Tage bei 4 °C.

Folgende Zusammensetzungen wurden als Stabilisatoren des Komplexes aus siRNA und Transfektionsreagenz verwendet:
1% (w/v) BSA
5% (w/v) BSA
DMEM mit Serum

Zur Kontrolle wurde eine Transfektion mit frisch angesetzen Transfektionskomplexen in DMEM ohne Serum durchgeführt.

Das Ergebnis dieses Versuches ist in Abbildung 3 dargestellt. Dabei hat sich gezeigt, dass die Transfektionseffizienz nach Lagerung in BSA in etwa so gut bis leicht besser ist als mit frisch angesetzten Transfektionskomplexen. Bei der Lagerung in DMEM mit Serum hingegen zeigte sich eine deutlich schlechtere Transfektionseffizienz, insbesondere dann, wenn mit geringen siRNA Konzentrationen (weniger als 5 nM) gearbeitet wurde.

### Beispiel 4:

Stabilisierung eines Komplexes von Transfektionsreagenz und siRNA mit verschiedenen Zusammensetzungen bei einer Lagerung des Komplexes für 5 Tage bei 4 °C mit unterschiedlichen Konzentrationen an eingesetzter siRNA

Vorgegangen wurde wie in Beispiel 3 beschrieben, nur dass diesmal die Lagerung für 5 anstelle von 2 Tagen bei 4 °C durchgeführt wurde und auf eine Kontrolle mit frisch angesetzten Transfektionskomplexen verzichtet wurde.

Das Ergebnis dieses Versuches ist in Abbildung 4 dargestellt. Abbildung 4 zeigt, dass die Transfektionseffizienz bei 1% und 5% BSA als Stabilisator nach 5 Tagen unverändert hoch geblieben ist, so dass sich keine negativen Auswirkungen der Lagerung auf die Transfektionseffizienz zeigen. Dahingegen lässt sich keine erfolgreiche Transfektion mehr durchführen, wenn der Komplex aus siRNA und Transfektionsreagenz für 5 Tage bei 4 °C in DMEM mit Serum gelagert worden ist.

### Beispiel 5:

Trocknung eines Komplexes von Transfektionsreagenz und siRNA ohne stabilisierende Zusammensetzung

Vorgegangen wurde wie in Beispiel 1 beschrieben, nur dass diesmal als siRNA ein Gemisch aus Hs_ubb und Hs_plk1 siRNAs verwendet wurde. Diese beiden siRNAs sorgen für das Ausschalten zweier verschiedener Gene in einer eukaryotischen Zelle für die Induktion des Zelltodes. Sie werden Cell Death Control siRNA. Es wurden die Mengen 6,6 nM, 16,6 nM und 33,3 nM siRNA eingesetzt. Als Transfektionsreagenz wurden 0,75 und 1,5 µl HiPerFect (QIAGEN) verwendet.

Anders als in Beispiel 1 beschrieben wurde in diesem Beispiel keine den Komplex aus Transfektionsreagenz und siRNA stabilisierende Zusammensetzung eingesetzt, sondern nur Transfektionsreagenz und siRNA in Puffer HBS. Der Komplex wurde über Nacht getrocknet und anschließend wieder in 100 µl Wasser aufgenommen. Zum Vergleich wurde wieder als Nullkontrolle die NSC siRNA (Allstar neg.) verwendet, die keinen Silencing-Effekt in Zellen ausübt.

Die Transfektion wurde durchgeführt wie in Beispiel 1 beschrieben. Als Kontrolle wurde auch mit frisch angesetzten Komplexen aus Transfektionsreagenz und siRNA transfiziert, die zuvor nicht getrocknet worden waren.

3 Tage nach der Transfektion wurde in allen Ansätzen die Zahl der überlebenden Zellen gemäß dem Protokoll des CellTiter Glo Luminescent Cell Viability Assays (Promega) bestimmt. Je mehr lebende Zellen im Ansatz vorhanden sind, desto höher sind die messbaren Lichteinheiten (Lumineszenz).

Abbildung 5 zeigt, dass nur in den frisch angesetzten Transfektionsansätzen, die zuvor nicht getrocknet worden waren, durch die Cell Death Control siRNA der Zelltod induziert worden ist, nicht jedoch in den Ansätzen, in denen der Komplex aus siRNA und Transfektionsreagenz zuvor getrocknet worden ist. Dies zeigt, dass die Trocknung ohne stabilisierende Zusammensetzung dazu führt, dass keine erfolgreiche Transfektion mehr durchgeführt werden kann.

### Beispiel 6:

Stabilisierung eines Komplexes von Transfektionsreagenz und siRNA mit stabilisierender Zusammensetzung durch Trocknung und Lagerung des Komplexes für 2 Monate bei 4 °C, Raumtemperatur und 37 °C

Vorgegangen wurde wie in Beispiel 1 beschrieben, nur dass als stabilisierende Zusammensetzung nur HBS-Puffer mit 1% (w/v) BSA und 0,1 M Saccharose in einem Volumen von 12,5 µl verwendet wurde. In diesem Experiment wurden zwei verschiedene siRNAs verwendet, die gegen das Transkript von WASL gerichtet sind, WASL_6 und WASL_9. Von WASL_6 ist bekannt, dass sie weniger funktionell ist als WASL_9. Zusätzlich wurde als Negativkontrolle die NSC siRNA eingesetzt. Als Zelllinien wurden diesmal zusätzlich zu HeLa S3 die Zelllinien MCF-7 und HepG2 verwendet.

Die Komplexe aus Transfektionsreagenz und siRNA wurden mit der stabilisierenden Zusammensetzung zusammengegeben und über Nacht im Vakuum getrocknet. Anschließend wurde der getrocknete Komplex für 2 Monate bei 4 °C, Raumtemperatur oder 37 °C gelagert und anschließend wieder in 12,5 µl Wasser aufgenommen, bevor die unterschiedlichen Zelllinien direkt transfiziert wurden.

Als Primer für die quantitative RT-PCR wurden WASLF und WASLR verwendet.

Zur Kontrolle wurden frisch angesetzte Transfektionskomplexe transfiziert, die weder getrocknet noch gelagert worden waren.

Das Ergebnis dieses Versuches ist in Abbildung 6 dargestellt. Dabei zeigte sich, dass in allen drei Zelllinien vergleichbare Ergebnisse erzielt worden waren. Die für zwei Monate gelagerten Komplexe mit der stabilisierenden Zusammensetzung waren in allen Fällen geeignet, um nach der Lagerung eine erfolgreiche Transfektion durchzuführen. Dabei zeigte sich, dass die Effizienz der Transfektion bei einer Lagerung bei 4 °C bei allen drei Zelllinien identische Ergebnisse brachte wie die Transfektion von frisch angesetzten Komplexen. Bei einer Lagerung bei Raumtemperatur war die Effizienz nur minimal schlechter als bei einer Lagerung bei 4 °C, auch eine Lagerung bei 37 °C für 2 Monate ergab noch eine sehr effiziente Transfektion, die jedoch etwas weniger effizient war verglichen mit der Lagerung bei 4 °C und bei Raumtemperatur.

Wird ein Komplex aus Transfektionsreagenz und siRNA in Anwesenheit einer Mischung aus BSA und Saccharose getrocknet, so ist der dadurch so stabilisiert, dass die Transfektionseffizienz nach 2 Monaten Lagerung nicht reduziert ist. Im Gegensatz dazu hat das Beispiel 5 gezeigt, dass die Transfektionseffizienz gegen Null geht, wenn der Komplex aus Transfektionsreagenz und siRNA ohne Anwesenheit einer Mischung aus Protein und Saccharid getrocknet wird.

### Beispiel 7:

Stabilisierung eines Komplexes von Transfektionsreagenz und siRNA mit stabilisierender Zusammensetzung nach mehreren Einfrier- und Auftauzyklen

Vorgegangen wurde wie in Beispiel 1 beschrieben, nur dass diesmal zusätzlich zu HeLa S3 Zellen auch MCF-7 Zellen zur Transfektion verwendet wurden und dass als stabilisierende Zusammensetzung HBS-Puffer mit 1% (w/v) BSA und 0,1 M Saccharose verwendet wurde. Die Komplexe aus Transfektionsreagenz, siRNA und stabilisierender Zusammensetzung wurden für 1 Stunde bei -20 °C eingefroren und anschließend für 1 Stunde bei Raumtemperatur aufgetaut. Dieser Zyklus wurde dreimal bzw. fünfmal wiederholt. Zur Kontrolle wurden frisch angesetzte Transfektionskomplexe transfiziert, die nicht eingefroren worden waren.

Das Ergebnis dieses Versuches ist in Abbildung 7 gezeigt. Dabei stellte sich heraus, dass ein mehrmaliges Einfrieren und Auftauen des Transfektionskomplexes mit der stabilisierenden Zusammensetzung eine ebenso effiziente Transfektion ermöglicht wie eine Transfektion mit einem frisch angesetzten Transfektionskomplex.

Dieser Versuch zeigt, dass sich der Komplex aus Transfektionsreagenz und Nukleinsäure problemlos in gefrorenem Zustand lagern lässt, wenn eine Zusammensetzung aus Protein und Saccharid den Komplex stabilisiert.

### Beispiel 8:

Stabilisierung eines Komplexes aus Transfektionsreagenz und siRNA mit stabilisierender Zusammensetzung und unterschiedlichen Transfektionsreagenzien

Vorgegangen wurde wie in Beispiel 1 beschrieben, nur dass diesmal zusätzlich zum Transfektionsreagenz HiPerFect das Transfektionsreagenz HT-Fect (QIAGEN) eingesetzt wurde. Als siRNA wurde diesmal CDC2 zusätzlich zu NSC eingesetzt, als Primer in der quantitativen RT-PCR wurden die Primer CDC2F und CDC2R verwendet.

Als stabilisierende Zusammensetzungen wurden verwendet:
Puffer A: 0,1 M Saccharose, 1% (w/v) BSA in HBS
Puffer B: 0,1 M Saccharose, 0,4% (w/v) BSA in HBS
Puffer C: 0,25 M Trehalose, 1 % (w/v) BSA in HBS
Puffer D: 0,1 M Trehalose, 0,4% (w/v) BSA in HBS

Der Komplex aus Transfektionsreagenz und siRNA wurde entweder getrocknet oder bei -20 °C nicht getrocknet in Anwesenheit der stabilisierenden Zusammensetzung gelagert. Vor der Transfektion wurden die Komplexe wieder in Wasser aufgenommen bzw. aufgetaut. Als Kontrolle wurden frisch angesetzte Komplexe aus Transfektionsreagenz und siRNA transfiziert.

Das Ergebnis dieses Versuches ist in Abbildung 8A für HT Fect und in Abbildung 8B für HiPerFect gezeigt.

Der Versuch hat gezeigt, dass sich die stabilisierende Eigenschaft der Zusammensetzung nicht nur auf das Transfektionsreagenz HiPerFect beschränkt, sondern dass auch weitere Transfektionsreagenzien mit Hilfe der Zusammensetzung in ihrem Komplex mit der Nukleinsäure stabilisiert werden können. Ferner hat der Versuch gezeigt, dass auch andere Saccharide als Saccharose in der Zusammensetzung verwendet werden können.

Abbildung 8A zeigt, dass die CDC2 mRNA nach der Transfektion mit HT Fect im vergleichbaren Maße herunterreguliert worden ist, wenn frisch transfiziert wurde oder wenn der Komplex aus Transfektionsreagenz und Nukleinsäure zuvor getrocknet wurde. Wurde der Komplex aus Transfektionsreagenz und Nukleinsäure jedoch bei -20 °C eingefroren, so ist nur noch die Zusammensetzung Puffer C geeignet, um nachfolgend eine erfolgreiche Transfektion mit HT Fect durchzuführen, bei den übrigen drei untersuchten Zusammensetzung war keine erfolgreiche Transfektion nach dem Einfrieren bei -20 °C festzustellen, wenn mit HT Fect transfiziert wurde.

Wurde hingegen mit HiPerfect transfiziert (Abbildung 8B), so zeigte sich, dass alle vier untersuchten Zusammensetzungen sowohl getrocknet als auch bei -20 °C gelagert eine zu frisch angesetzten Transfektionskomplexen vergleichbare Transfektionseffizienz ergaben. Dabei waren die Ergebnisse für Saccharose und für Trehalose vergleichbar.

Dieser Versuch hat gezeigt, dass andere Saccharide als Saccharose ebenfalls geeignet sind, um in der Mischung mit einem Protein einen Komplex aus Transfektionsreagenz und Nukleinsäure zu stabilisieren.

Weiter hat dieser Versuch gezeigt, dass die stabilisierende Wirkung der Zusammensetzung nicht auf ein einziges Transfektionsreagenz beschränkt ist, wobei der Versuch auch gezeigt hat, dass es Transfektionsreagenzien gibt, für die die stabilisierende Zusammensetzung geeigneter sein kann als für andere.

### Beispiel 9:

Stabilisierung eines Komplexes aus Transfektionsreagenz und Plasmid-DNA

2, 3 bzw. 4 µl HiPerfect wurden mit 400 ng Plasmid-DNA, welche ein offenes Leseraster für das Green Fluorescent Protein aufwies (pGFP) in 50 µl HBS Puffer zusammengegeben. In die Ansätze wurden zusätzlich als stabilisierende Zusammensetzung 0,1 M Sacchaorse und 1% (w/v) BSA gegeben (Premix).

Nach der Komplexbildung für 10 min bei Raumtemperatur wurden die Komplexe über Nacht im Vakuum getrocknet und im getrockneten Zustand bei 4 °C für 4 Stunden gelagert.Vor der Transfektion wurden die Komplexe in Wasser aufgenommen und transfiziert, wie in Beispiel 1 angegeben. Abweichend von Beispiel 1 wurde diesmal Plasmid-DNA und keine siRNA transfiziert und zusätzlich zu HeLa S3 Zellen wurden auch MCF-7 Zellen mit der Plasmid-DNA transfiziert. Zum Vergleich wurde die Plasmid-DNA auch im frischen Transfektionskomplex mit HiPerFect transfiziert (Standard).

Nach einer Inkubation des Transfektionsansatzes für 2 Tage bei 37 °C und 5 % CO₂ wurde die Anzahl der transfizierten Zellen mittels Fluoreszenz aktiviertem Zell-Sortierens (FACS) in einem FACSCalibur (Becton Dickinson) gemessen. Alle Zellen, die erfolgreich mit dem pGFP Plasmid transfiziert wurden, exprimieren das GFP, welches eine im FACS messbare Fluoreszenz emittiert.

In Abbildung 9 ist das Ergebnis der FACS Analyse dargestellt. Bei allen drei getesteten Konzentration an Transfektionsreagenz ist die Transfektionseffizienz in HeLa S3 Zellen sehr hoch und nahezu identisch, wenn die Transfektion nach Trocknung des Komplexes mit stabilisierender Zusammensetzung oder frisch ohne stabilisierende Zusammensetzung erfolgte.

Bei MCF-7 Zellen ist die Transfektionseffizienz ebenfalls sehr hoch, die Effizienz der Transfektion nach Trocknung ist nur geringfügig niedriger als beim frischen Ansatz.

Dieses Experiment zeigt, dass sich die stabilisierende Zusammensetzung nicht nur zur Stabilisierung von RNA mit einem Transfektionsreagenz eignet, sondern auch im gleichen Maße zur Stabilisierung von DNA mit einem Transfektionsreagenz.

## Patentansprüche

1. Verfahren zur Transfektion einer eukaryotischen Zelle, aufweisend die folgenden Verfahrensschritte:
a. In Kontakt Bringen einer Zusammensetzung, aufweisend mindestens ein Saccharid und mindestens ein Protein, sowie optional ein Salz und/oder eine pH-Wert stabilisierende Komponente, mit einer Nukleinsäure und einem Transfektionsreagenz;
b. Ausbilden eines Komplexes aus Nukleinsäure und Transfektionsreagenz unter Wechselwirkung mit der Zusammensetzung aus Schritt a;
c. Transfektion einer eukaryotischen Zelle mittels des Komplexes aus Schritt b.

2. Verfahren nach Anspruch 1, **dadurch gekennzeichnet, dass** nach Schritt b und vor Schritt c eine Lagerung des Komplexes erfolgt.

3. Verfahren nach Anspruch 2, **dadurch gekennzeichnet, dass** der Komplex zur Lagerung getrocknet wird und vor der Transfektion in Schritt c wieder in einer Flüssigkeit aufgenommen wird.

4. Verfahren nach Anspruch 3, **dadurch gekennzeichnet, dass** die Trocknung im Vakuum stattfindet.

5. Verfahren nach Anspruch 3, **dadurch gekennzeichnet, dass** die Trocknung bei Atmosphärendruck stattfindet.

6. Verfahren nach einem der Ansprüche 2 bis 5, **dadurch gekennzeichnet, dass** die Lagerung des Komplexes bei einer Temperatur von 2 °C bis 8 °C erfolgt.

7. Verfahren nach einem der Ansprüche 2 bis 5, **dadurch gekennzeichnet, dass** die Lagerung des Komplexes bei einer Temperatur unter 0°C erfolgt und der Komplex zur Durchführung der Transfektion in Schritt c als Flüssigkeit vorliegt.

8. Verfahren nach einem der Ansprüche 2 bis 5, **dadurch gekennzeichnet, dass** die Lagerung des Komplexes bei Raumtemperatur erfolgt.

9. Verwendung einer Zusammensetzung zur Stabilisierung eines Komplexes aus einer Nukleinsäure und einem Transfektionsreagenz, **dadurch gekennzeichnet, dass** die Zusammensetzung mindestens ein Saccharid und mindestens ein Protein aufweist, sowie optional ein Salz und/oder eine pH-Wert stabilisierende Komponente.

10. Zusammensetzung zur Lagerung eines Komplexes aus einer Nukleinsäure und einem Transfektionsreagenz, **dadurch gekennzeichnet, dass** sie mindestens ein Saccharid und mindestens ein Protein sowie eine Nukleinsäure und ein Transfektionsreagenz aufweist, sowie optional ein Salz und/oder eine pH-Wert stabilisierende Komponente.

11. Zusammensetzung nach Anspruch 10, **dadurch gekennzeichnet, dass** es sich beim Protein um BSA handelt.

12. Zusammensetzung nach Anspruch 11, **dadurch gekennzeichnet, dass** das BSA in der Zusammensetzung einen Gewichtsanteil von 0, 1 % bis 5% aufweist.

13. Zusammensetzung nach einem der Ansprüche 10 bis 12, **dadurch gekennzeichnet, dass** es sich bei dem Saccharid um Trehalose, Saccharose oder ein Gemisch von beiden handelt.

14. Zusammensetzung nach Anspruch 13, **dadurch gekennzeichnet, dass** es sich bei dem Saccharid um Saccharose handelt.

15. Zusammensetzung nach Anspruch 13 oder 14, **dadurch gekennzeichnet, dass** das Saccharid in einer Konzentration von 0,01 M bis 2 M vorliegt.
